# EUROPEAN PATENT SPECIFICATION

(11) **EP 4 208 092 B1**
(45) Date of publication and mention of the grant of the patent: **04.12.2024**
(21) Application number: 21798161.2
(22) Date of filing: 01.09.2021
(51) Int. Cl.: A61B 5/1495, A61B 5/20, A61B 5/1455, A61B 5/00, A61B 5/1459

(54) **ACUTE KIDNEY INJURY MONITORING**
ÜBERWACHUNG AKUTER NIERENVERLETZUNGEN
SURVEILLANCE D'UNE INSUFFISANCE RÉNALE AIGUË

(30) Priority: 04.09.2020 US 202063074763 P; 24.08.2021 US 202117410834
(43) Date of publication of application: 12.07.2023
(73) Proprietor: Covidien LP, Mansfield, MA 02048 (US)
(72) Inventor: DOVE, Jacob D., Boulder, Colorado 80301 (US); AASMUL, Soren, 2300 Copenhagen (DK); SMITH, William S., Boulder, Colorado 80301 (US); MILLER, David J., Boulder, Colorado 80301 (US); KRISTENSEN, Jesper Svenning, 2300 Copenhagen (DK)
(74) Representative: Marks & Clerk LLP
(86) International application number: PCT/US2021/048708
(87) International publication number: WO 2022/051394

(56) References cited:
- WO-A1-2017/070155
- US-A1- 2019 069 831

## Description

### TECHNICAL FIELD

This disclosure relates to patient monitoring.

### BACKGROUND

Medical devices, such as catheters, may be used to assist a patient in voiding their bladder. In some instances, such catheters may be used during and/or after surgery. In the case of using a catheter to assist a patient in voiding their bladder, a Foley catheter is a type of catheter that may be used for longer time periods than a non-Foley catheter. Some Foley catheters are constructed of silicon rubber and include an anchoring member, which may be an inflatable balloon, that may be inflated in a patient's bladder to serve as an anchor so a proximal end of the catheter does not slip out of the patient's bladder.

WO 2017/070155 A1 discloses a device for insertion into a patient, the device comprising a shaft and a light delivery element. The shaft comprises a proximal end and a distal end, and the light delivery element is positioned proximate the distal end of the shaft. The device is configured to repeatedly adjust light delivered by the light delivery elements. US 2019/069831 A1 discloses an oxygen-sensing assembly for attachment to a urinary catheter, the assembly including a housing having a flow pathway extending between an inlet end and an outlet end thereof, and an oxygen sensor in operable communication with the flow pathway of the housing. The oxygen sensor is configured to detect oxygen levels of a fluid flowing through the flow pathway, a flowrate sensor configured to detect a flowrate of the fluid flowing through the flow pathway, and a temperature sensor configured to detect a temperature of the fluid flowing through the flow pathway.

### SUMMARY

The invention is best summarized in accordance with the appended claims.

### BRIEF DESCRIPTION OF DRAWINGS

FIG. 1 is a diagram illustrating an example catheter.
FIG. 2 is a diagram illustrating an example cross-sectional view of the catheter of FIG. 1, the cross-sections being taken along lines 2-2 of FIG. 1.
FIG. 3 is a block diagram of an example external device that may be used with a medical device according to example techniques of this disclosure.
FIG. 4 is a graph illustrating pO₂ measurements of water that was initially set to ~43 mmHg and allowed to flow through a silicone Foley catheter at different flow rates.
FIG. 5 is a conceptual diagram of an example fiberoptic system according to the techniques of this disclosure.
FIG. 6 is a conceptual diagram illustrating the proximal portion of an example fiberoptic system within a closed lumen of an example catheter.
FIG. 7 is a conceptual diagram illustrating the proximal portion of an example fiberoptic system including a shield.
FIG. 8 is a flowchart illustrating example monitoring techniques of this disclosure.

### DETAILED DESCRIPTION

Acute kidney injury (AKI) is a complication that may occur after some medical procedures, such as some cardiac surgeries, e.g., coronary artery bypass grafting (CABG). AKI may also occur after other surgeries that are lengthy and involve significant blood loss or fluid shifts. For example, a surgery patient's body may alter where their blood is directed which may lead to hypoxia of a kidney. A cause of surgery-associated AKI is hypoxia of the kidneys, which may cause an ischemia reperfusion injury in a kidney of the patient. This ischemia reperfusion injury may cause degradation of renal function of the patient. The degradation of renal function may cause an accumulation of waste products in the bloodstream, which may delay the patient's recovery from the surgery and lead to more extended hospital stays and may even lead to further complications.

The present disclosure describes example devices that are configured to monitor kidney function of patients, such as patients who are undergoing or who have undergone such surgeries, which may help reduce occurrences of AKI by providing clinicians with an assessment of the risk that a specific patient may develop AKI. This may facilitate a clinician intervening prior to the patient developing AKI. For example, a clinician may initiate or make changes to hemodynamic management (e.g., blood pressure management, fluid management, blood transfusions, and the like), make changes to cardiopulmonary bypass machine settings, or avoid providing nephrotoxic drugs. Post operatively, a clinician may intervene with a Kidney Disease: Improving Global Outcomes (KDIGO) bundle or an AKI care bundle, which may be predetermined set of guidelines and practices for the clinician to follow. The devices may include or be configured to accept one or more sensors configured to sense different parameters of a fluid of interest, such as urine in the case of kidney function monitoring. While urine, bladders, and AKI are primarily referred to herein to describe the example devices, in other examples, the devices may be used with other target locations in a patient, such as intravascular locations, and to monitor fluids of interest other than urine and/or other patient conditions other than kidney function.

While systemic vital signs like cardiac output, blood pressure, and hematocrit may be useful for monitoring the kidney function of a patient (also referred to herein as renal monitoring), it may also be useful to monitor the oxygenation status of the kidneys in order to limit, reduce the severity of, or even prevent the risk of AKI. Accurate monitoring of the oxygenation status of the kidneys can be challenging due to the inaccessibility of the kidneys. Near-Infrared spectroscopy (NIRS) measures regional oximetry, and has some utility in babies and relatively slender adults in measuring oxygenation of the kidneys, but may not have the depth of penetration and specificity required for some patients.

The present disclosure describes example medical devices, such as catheters, sensors, fiberoptic systems and external devices, that are configured to sense and/or monitor kidney function of patients, such as patients who are undergoing or who have undergone surgeries or other medical procedures, which may help reduce occurrences of AKI. In some examples, the medical device (e.g., catheter) includes an oxygen sensing element configured to sense an amount of dissolved oxygen in a fluid, such as urine in the case of kidney function monitoring. In some examples, the oxygen sensing element may not be a part of the medical device, but be part of a separated device (e.g., a fiberoptic system) that is insertable into a lumen of a catheter, such as a three or more lumen Foley catheter. In either example, the oxygen sensing element may also be configured to provide a signal indicative of the amount of dissolved oxygen in the fluid to processing circuitry. The oxygen sensing element may also be referred to as an oxygen sensor or as oxygen sensing circuitry in some examples, though the sensing circuitry can include non-electrical components, such as one or more fiber-optic components.

While urine, bladders, and AKI are primarily referred to herein to describe the example medical devices, in other examples, the medical devices may be used with other target locations in a patient, such as intravascular locations, and to monitor fluids of interest other than urine and/or other patient conditions other than kidney function. In addition, while catheters are primarily referred to herein, in other examples, the medical device can have another configuration. As discussed in further detail below, in some examples, the oxygen sensing element may include a dissolved oxygen sensor configured to sense an amount of oxygen dissolved in the urine (e.g., urinary oxygen tension (uPO₂ or PuO₂)) in the bladder or in the catheter, from which a clinician or a device may be able to determine an oxygenation status of the one or both kidneys of the patient.

While this disclosure is primarily focused on sensing an amount of dissolved oxygen in a fluid and sensing a temperature of the fluid, other parameters of interest may be sensed by a medical device, such as a catheter. These other parameters of interest may include, but are not limited to, any one or more of urine flow rate, urine concentration, urine electrical conductivity, urine specific gravity, urine biomarkers, amount of dissolved carbon dioxide in the urine, urine pH, bladder or abdominal pressure, urine color, urine turbidity, urine creatinine, urine electrical conductivity, urine sodium, or motion from an accelerometer or other motion sensor. In some cases, it may be desirable to sense one or more of these parameters relatively close to the kidneys as possible because when sensors are positioned further away from the kidneys, the risk of introducing noise or losing signal strength increases and/or the risk of the concentration or integrity of a substance of interest in the fluid of interest (e.g., urine) changing prior to being sensed by the sensor may increase.

In the case of a Foley catheter, it may be desirable to sense the amount of dissolved oxygen in the fluid, the temperature of the fluid, and/or one or more of the other parameters listed above at a proximal portion of the Foley catheter (e.g., in the bladder of the patient). However, placing these sensors at the proximal portion of the catheter may increase the size and stiffness of the catheter and, as a result, may undermine the patient comfort or deliverability of the catheter. By design, a Foley catheter is configured to be relatively small and flexible, such that it can be inserted through the urethra and into the bladder of a patient. If a Foley catheter were stiffer or include sensors disposed on an outer surface of the catheter, then it may be more difficult to comfortably insert the catheter into the bladder of the patient.

As used herein, "sense" may include detect and/or measure." As used herein, "proximal" is used as defined in Section 3.1.4 of ASTM F623-19, Standard Performance Specification for Foley Catheter. That is, a proximal end of a catheter is the end closest to the patient when the catheter is being used by the patient. The distal end is therefore the end furthest from the patient. In some examples, "block" may mean completely prevent or partially prevent (e.g., effectively prevent), such as by blocking, restricting, inhibiting, impeding, or hindering. For example, to block ingress of a fluid into the lumen may mean that the fluid does not enter the lumen or is restricted, inhibited, impeded or hindered from entering the lumen.

The amount of dissolved oxygen in a patient's urine may be indicative of kidney function or kidney health. For example, dissolved oxygen in a patient's urine in the bladder may correlate to perfusion and/or oxygenation of the kidneys, which is indicative of kidney performance. However, dissolved oxygen can be relatively difficult to measure. One way to measure dissolved oxygen is by fluorescence or luminescence lifetime sensor(s). For example, an oxygen sensing element may be a portion of or all of a fluorescence or luminescence lifetime sensor. The oxygen sensing element may utilize a light which may originate from processing circuitry and sense the decay of glow from the light in a fluid, which may be indicative of the level of oxygen in the fluid. To more accurately measure the level of oxygen in a patient's urine, it may be desirable to take the measurement prior to any significant modification in the oxygen content in the urine, e.g., as close to the kidneys as possible. However, it may not be feasible to place all of a dissolved oxygen sensor at the proximal end of the catheter as doing so may increase cost, size, and decrease flexibility of the catheter.

Some Foley catheters include an elongated body made from a silicone rubber that is relatively permeable to oxygen. Thus, as a fluid flows through a drainage lumen of the Foley catheter from a proximal fluid opening to the drainage lumen to a distal fluid opening to the drainage lumen, some oxygen may permeate from the surrounding environment through the walls of the elongated body into urine in the drainage lumen or dissipate through the walls of the elongated body and into a surrounding environment, or vice versa. For example, urine oxygenation for some patients may be 10 millimeters of mercury (mmHg) to 50mmHg, which is substantially lower than the atmospheric level of about 150mmHg, creating a gradient that can drive atmospheric oxygen into the catheter.

In accordance with examples of this disclosure, a catheter assembly includes a catheter (e.g., a Foley catheter) defining a plurality of lumens, a first lumen being configured to receive a fluid and a second lumen configured to receive or house a sensor, such an oxygen sensing element. The catheter assembly further includes the oxygen sensing element configured to, while in the second lumen, sense an amount of dissolved oxygen in the fluid (e.g., urine) external to the second lumen. This may enable the oxygen sensing element to sense the dissolved oxygen in urine relatively close to a bladder of a patient or in the bladder of the patient without directly contacting the fluid. That is, the catheter is configured to block ingress of the fluid into the second lumen while the oxygen sensing element senses the amount of dissolved oxygen. This may help maintain the integrity of the oxygen sensing element.

In some examples, the oxygen sensing element is separate from and configured to be introduced into the second lumen of the catheter, which may enable the catheter to remain relatively flexible, e.g., compared to examples in which the oxygen sensing element and associated wires or fiber optic elements is integrated into the catheter. The flexibility may help maintain the deliverability of the Foley catheter proximal end to the bladder.

The oxygen sensing element is configured to sense an amount of dissolved oxygen in a fluid (e.g., urine) and provide a signal indicative of the amount of dissolved oxygen in the fluid to processing circuitry. The oxygen sensing element may be located in a proximal portion of a lumen of a catheter. The processing circuitry may be located at a distal portion of the lumen or distal to a distal end of the lumen.

The second lumen of the catheter may also be referred to as a sensor lumen. In some examples, the catheter is a Foley catheter, which defines a drainage lumen and a sensor lumen. The drainage lumen is configured to facilitate the flow of the fluid from a first fluid opening at a proximal end of the catheter to a second fluid opening at a distal end of the opening, e.g., from a bladder to a collection container outside of a patient. In contrast , and according to the invention, the sensor lumen does not include an opening configured to receive the fluid, e.g., has a closed proximal end, and is configured to receive or house an oxygen sensing element. When the oxygen sensing element is located in a proximal portion of the second lumen, the oxygen sensing element can sense an amount of dissolved oxygen in a fluid external to the sensor lumen. In some examples, the Foley catheter further defines a third lumen, referred to as an anchoring lumen, which is associated with an anchoring mechanism proximate to a proximal end of the Foley catheter and is configured to facilitate deployment of the anchoring mechanism to anchor the Foley catheter within a patient. For example, the anchoring lumen can be configured to receive an inflation fluid to inflate a balloon anchoring mechanism within a bladder of patient.

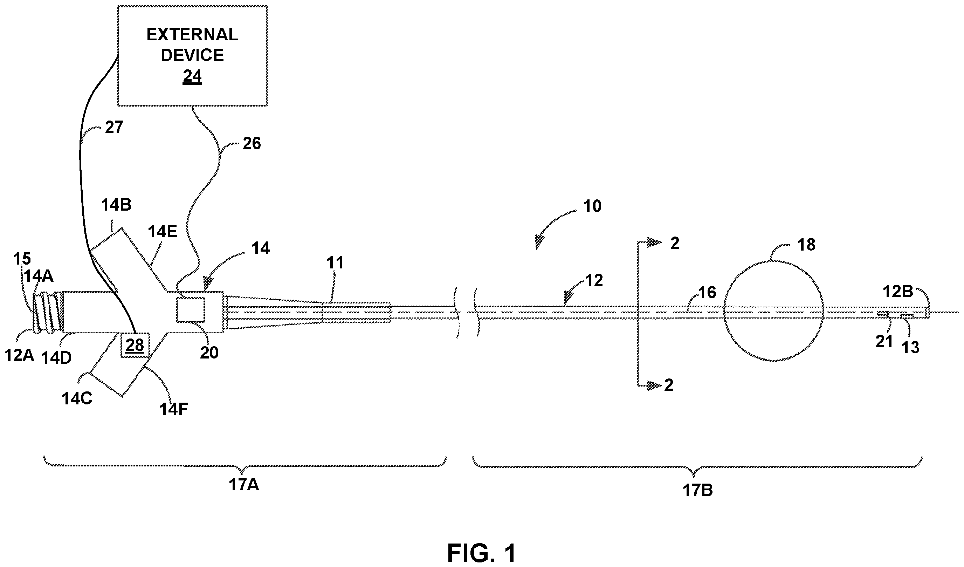

FIG. 1 is a conceptual side elevation view of an example catheter 10, which includes elongated body 12, hub 14, and anchoring member 18. In some examples, catheter 10 is a Foley catheter. While a Foley catheter and its intended use are primarily referred to herein to describe catheter 10, in other examples, catheter 10 can be used for other purposes, such as to drain wounds or for intravascular monitoring or medical procedures.

Catheter 10 includes a distal portion 17A and a proximal portion 17B. Distal portion 17A includes a distal end 12A of elongated body 12 and is intended to be external to a patient's body when in use, while proximal portion 17B includes a proximal end 12B of elongated body 12 and is intended to be internal to a patient's body when in use. For example, when proximal portion 17B is positioned within a patient, e.g., such that proximal end 12B of elongated body 12 is within the patient's bladder, distal portion 17A may remain outside of the body of the patient.

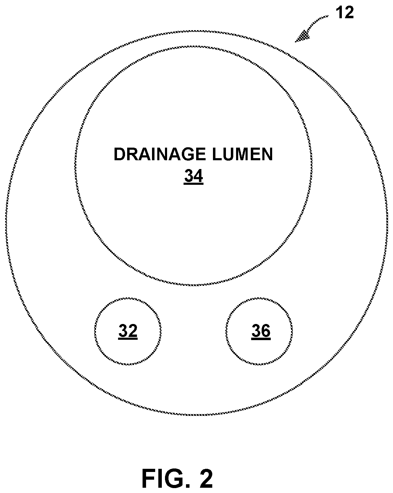

Elongated body 12 is a structure (e.g., a tubular structure) that extends from distal end 12A to proximal end 12B and defines one or more inner lumens. In the example shown in FIGS. 1-2, elongated body 12 defines lumen 32, drainage lumen 34, and anchoring lumen 36 (shown in FIG. 2). In some examples, drainage lumen 34 is configured to drain a fluid from a target site, such as a bladder. In other examples, drainage lumen 34 may be used for any other suitable purpose, such as to deliver a substance or another medical device to a target site within a patient. Drainage lumen 34 may extend from proximal fluid opening 13 to distal fluid opening 14A. Both proximal fluid opening 13 and distal fluid opening 14A may be fluidically coupled to drainage lumen 34, such that a fluid may flow from one of fluid opening 13 or fluid opening 14A to the other of fluid opening 13 or fluid opening 14A through drainage lumen 34. Fluid opening 13 and fluid opening 14A may also be referred to as drainage openings.

In some examples, lumen 32 (shown in FIG. 2) may be configured to receive or house sensor 21. In this manner, lumen 32 may be referred to as a sensor lumen. Sensor 21 may include an oxygen sensing element and/or a temperature sensor. In some examples, lumen 32 extends from distal opening 14C to a location proximate to anchoring member 18 (e.g., distal to or proximal to anchoring member 18). In some examples, lumen 32 is closed on the proximal portion of lumen 32 such that fluid may not flow into lumen 32 from a bladder of a patient when proximal end 12B is inserted into the bladder of the patient. In addition, and in accordance with the invention, lumen 32 is closed except for distal opening 14C. Elongated body 12 is configured to block ingress of the fluid into lumen 32 while the oxygen sensing element of sensor 21 senses the amount of dissolved oxygen in a fluid external to elongated body 12. In this way, elongated body 12 is configured to substantially block (e.g., prevent or nearly prevent to the extent permitted by manufacturing tolerances) the oxygen sensing element of sensor 21 from directly contacting the fluid, e.g., urine in a bladder of a patient. In these examples, however, elongated body 12 is relatively permeable to oxygen, thereby enabling oxygen sensing element 21 to generate a signal indicative of an amount of dissolved oxygen in the fluid without being in direct contact with the fluid. That is, oxygen sensing element 21 may sense the amount of dissolved oxygen in the fluid external to elongated body 12 despite not being in direct contact with the fluid because the oxygen may permeate from the fluid through the wall of elongated body 12 to oxygen sensing element 21 in lumen 32.

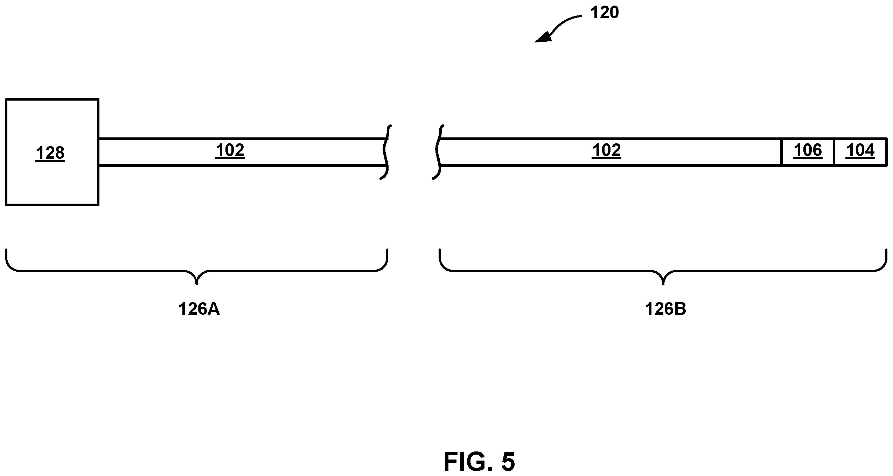

In some examples, sensor 21 is part of a fiberoptic system including an optical fiber (discussed further hereinafter with respect to FIGS. 5-8). For example, a proximal end of the fiberoptic system may be configured to be introduced into lumen 32 via distal opening 14C. In some examples, the fiberoptic system may include processing circuitry 28 which may be located on distal portion 17A of elongated body 12, or distal to distal end 12A. Processing circuitry 28 may include optical, optoelectrical, and/or electrical components and may be configured to determine an amount of dissolved oxygen in a fluid based on a signal received from sensor 21 and/or determine a temperature of the fluid based on a signal received from sensor 21. For example, sensor 21 may include a fluorescence or luminescence lifetime sensor(s) and sensor 21 may receive light from processing circuitry 28, may focus that light towards a fluid and sense the decay of the glow caused by the light. In some examples, the fiberoptic system may not include processing circuitry 28.

In some examples, the fiberoptic system may be coupled to external device 24 and be configured to provide a signal indicative of the amount of dissolved oxygen in the fluid to processing circuitry of external device 24 via connection 27. External device 24 may be a computing device, such as a workstation, a desktop computer, a laptop computer, a smart phone, a tablet, a server or any other type of computing device that may be configured to receive, process and/or display sensor data. In some examples, the signal may be a sensed signal from sensor 21. In other examples, the signal may be a signal from processing circuitry 28. Connection 27 may be an electrical, optical, wireless or other connection.

Proximal portion 17B of catheter 10 comprises anchoring member 18, fluid opening 13, and sensor 21. In some examples, sensor 21 is received or housed within lumen 32. Fluid opening 13 may be positioned on the surface of elongated body 12 between anchoring member 18 and the proximal end 12B (as shown) or may be positioned at the proximal end 12B.

Anchoring member 18 may include any suitable structure configured to expand from a relatively low profile state to an expanded state in which anchoring member 18 may engage with tissue of a patient (e.g., inside a bladder) to help secure and prevent movement of proximal portion 17B out of the body of the patient. For example, anchoring member 18 can include an anchor balloon or other expandable structure. When inflated or deployed, anchoring member 18 may function to anchor catheter 10 to the patient, for example, within the patient's bladder. In this manner, the portion of catheter 10 on the proximal side of anchoring member 18 may not slip out of the patient's bladder.

Anchoring lumen 36 (shown in FIG. 2) may be configured to transport a fluid, such as sterile water or saline, or a gas, such as air, from distal opening 14B to anchoring member 18. For example, an inflation device (not shown) may pump fluid or gas into anchoring lumen 36 through distal opening 14B into anchoring member 18 such that anchoring member 18 is inflated to a size suitable to anchor catheter 10 within the patient's bladder. In examples in which anchoring member 18 does not include an expandable balloon, anchoring lumen 36 may be configured to receive a deployment mechanism (e.g., a pull wire or a push wire) for deploying an expandable structure anchoring member 18 and hub 14 may comprise distal fluid opening 14A, distal opening 14C and a distal opening 14B via which a clinician may access the deployment mechanism.

In some examples, such as examples in which catheter 10 is a Foley catheter, elongated body 12 has a suitable length for accessing the bladder of a patient through the urethra. The length may be measured along central longitudinal axis 16 of elongated body 12. In some examples, elongated body 12 may have an outer diameter of about 12 French to about 14 French, but other dimensions may be used in other examples. Distal portion 17A and proximal portion 17B of elongated body 12 may each have any suitable length.

In the example shown in FIG. 1, distal end 12A of elongated body 12 is received within hub 14 and is mechanically connected to hub 14 via an adhesive, welding, or another suitable technique or combination of techniques. Hub 14 is positioned at a distal end of elongated body 12 and defines an opening through which the one or more inner lumens (e.g., lumen 32, drainage lumen 34 and anchoring lumen 36, shown in FIG. 2) of elongated body 12 may be accessed and, in accordance with the invention, closed. While hub 14 is shown in FIG. 1 as having three arms, 14D, 14E and 14F, hub 14 may have any suitable number of arms, which may, in some examples, depend on the number of inner lumens defined by elongated body 12. For example, each arm may be fluidically coupled to a respective inner lumen of elongated body 12. In the example of FIG. 1, hub 14 comprises a distal fluid opening 14A, which is fluidically coupled to drainage lumen 34, a distal opening 14B, which is fluidically coupled to anchoring lumen 36, and distal opening 14C which is fluidically coupled to lumen 32 (shown in FIG. 2) of elongated body 12. In examples in which anchoring member 18 does not include an expandable balloon, anchoring lumen 36 may be configured to receive a deployment mechanism (e.g., a pull wire or a push wire) for deploying an expandable structure anchoring member 18.

In examples in which catheter 10 is a Foley catheter, a fluid collection container (e.g., a urine bag) may be attached to distal fluid opening 14A for collecting urine draining from the patient's bladder. Distal opening 14B may be operable to connect to an inflation device to inflate anchoring member 18 positioned on proximal portion 17B of catheter 10. Anchoring member 18 may be uninflated or undeployed when not in use. Hub 14 may include connectors, such as connector 15, for connecting to other devices, such as the fluid collection container and the inflation source. Distal opening 14C may be operable to receive a fiberoptic system that may include sensor 21, which may be an oxygen sensor. In some examples, catheter 10 includes strain relief member 11, which may be a part of hub 14 or may be separate from hub 14.

In some examples, sensor 20 may be positioned on distal portion 17A, such as on hub 14. In some examples, sensor 20 is alternatively positioned distal to distal end 12A, such as on additional tubing or another structure connected to hub 14. Sensor 20 may be configured to sense a parameter of interest, in a fluid, such as urine. The fluid can be, for example, fluid in drainage lumen 34 or fluid received from drainage lumen 34.

Sensor 20 may be positioned on hub 14, as shown, or may be positioned elsewhere on distal portion 17A of elongated body 12 of catheter 10, or may be positioned distal to distal end 12A, e.g., on tubing connected to a fluid collection container (e.g., a urine bag) or the like. Sensor 20, may be one or more sensors that are relatively larger, require relatively more electrical, optoelectrical, or optical connections, than sensors that could be located on the proximal portion 17B. In some examples, sensor 20 may be configured to sense one or more of fluid output, flow rate, temperature, pressure, fluid concentration, amount of dissolved carbon dioxide in the fluid, turbidity, fluid pH, fluid color, fluid creatinine, motion, or other parameter of interest. In some examples, sensor 20 may not be included on catheter 10.

In some examples, sensor 20 is mechanically connected to elongated body 12 or another part of catheter 10 using any suitable technique, such as, but not limited to, an adhesive, welding, by being embedded in elongated body 12, via a crimping band or another suitable attachment mechanism or combination of attachment mechanisms. As discussed above, in some examples, sensor 20 is not mechanically connected to elongated body 12 or catheter 10, but is instead mechanically connected to a structure that is distal to a distal end of catheter 10, such as to tubing that extends between hub 14 and a fluid collection container.

Sensor 20 may be configured to communicate sensor data to external device 24. Sensor 20 may communicate sensor data to external device 24 via a connection 26. Connection 26 may be an electrical, optical, wireless or other connection.

Although sensor 20 and sensor 21 are shown in FIG. 1, in other examples, catheter 10 can include any suitable number of sensors on proximal portion 17B and/or any suitable number of sensors on distal portion 17A, where the sensors on proximal portion 17B sense the same or different parameters and the sensors on distal portion 17A sense the same or different parameters. In addition, some or all of the sensors on proximal portion 17B may sense the same or different parameters as the sensors on distal portion 17A. For example, in the case where sensors on the distal portion may be temperature dependent, it may be desirable to sense temperature both on the proximal portion 17B and the distal portion 17A.

Elongated body 12 may be structurally configured to be relatively flexible, pushable, and relatively kink- and buckle- resistant, so that it may resist buckling when a pushing force is applied to a relatively distal portion of the medical device to advance the elongated body proximally through the urethra and into the bladder. Kinking and/or buckling of elongated body 12 may hinder a clinician's efforts to push the elongated body proximally.

In some examples, at least a portion of an outer surface of elongated body 12 includes one or more coatings, such as an anti-microbial coating, and/or a lubricating coating. The lubricating coating may be configured to reduce static friction and/ kinetic friction between elongated body 12 and tissue of the patient as elongated body 12 is advanced through the urethra.

FIG. 2 is a diagram illustrating an example cross-section of elongated body 12 of catheter 10, where the cross-section is taken along line 2-2 in FIG. 1 in a direction orthogonal to central longitudinal axis 16. FIG. 2 depicts a cross section of elongated body 12, which defines lumen 32, drainage lumen 34, and anchoring lumen 36. While lumen 32, drainage lumen 34, and anchoring lumen 36 are shown as circular in cross-section, they may have any suitable cross-sectional shape in other examples.

Elongated body 12 may define any suitable number of lumens. For example, although one anchoring lumen 36 is shown in FIG. 2, in other examples, elongated body 12 can define a plurality of anchoring lumens 36, e.g., that are distributed around lumen 32 or drainage lumen 34. As another example, anchoring member 18 may be an expandable structure that is not an inflatable balloon. In such examples, anchoring lumen 36 may be replaced by or house a deployment mechanism which may permit a clinician to expand the expandable structure. For example, anchoring lumen 36 may be replaced by or house a mechanical device that may be pushed and pulled separately from the catheter 10 by a clinician to expand or retract the expandable structure.

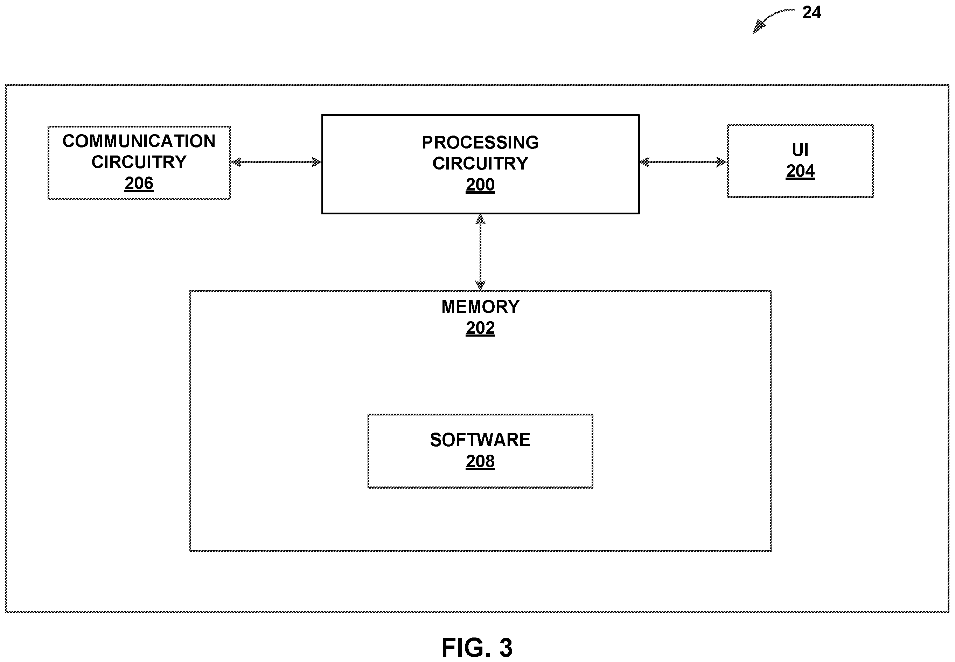

FIG. 3 is a functional block diagram illustrating an example of an external device 24 configured to communicate with sensor 20, receive information from sensor 20. In some examples, external device 24 also is configured to communicate with or receive information from sensor 21 or processing circuitry 28. In the example of FIG. 3, external device 24 includes processing circuitry 200, memory 202, user interface (UI) 204, and communication circuitry 206. External device 24 may be a dedicated hardware device with dedicated software for the reading sensor data. Alternatively, external device 24 may be an off-the-shelf computing device, e.g., a desktop computer, a laptop computer, a tablet, or a smartphone running a mobile application that enables external device 24 to read sensor data from sensor 20, sensor 21, or processing circuitry 28.

In some examples, a user of external device 24 may be clinician, physician, or heath care giver. In some examples, a user uses external device 24 to monitor a patient's kidney function. In some examples, the user may interact with external device 24 via UI 204, which may include a display to present a graphical user interface to the user and/or sound generating circuitry configured to generate audio output, and a keypad or another mechanism (such as a touch sensitive screen) configured to receive input from the user. External device 24 may communicate with sensor 20, sensor 21, or processing circuitry 28 using wired, wireless or optical methods through communication circuitry 206. For example, processing circuitry 200 of external device 24 may process sensor data from sensor 20 or sensor 21.

Processing circuitry 200 may include any combination of integrated circuitry, discrete logic circuity, analog circuitry, such as one or more microprocessors, digital signal processors (DSPs), application specific integrated circuits (ASICs), or field-programmable gate arrays (FPGAs). In some examples, processing circuitry 200 may include multiple components, such as any combination of one or more microprocessors, one or more DSPs, one or more ASICs, or one or more FPGAs, as well as other discrete or integrated logic circuitry, and/or analog circuitry.

Memory 202 may store program instructions, such as software 208, which may include one or more program modules, which are executable by processing circuitry 200. When executed by processing circuitry 200, such program instructions may cause processing circuitry 200, and external device 24 to provide the functionality ascribed to them herein. The program instructions may be embodied in software and/or firmware. Memory 202 may include any volatile, non-volatile, magnetic, optical, or electrical media, such as a random access memory (RAM), read-only memory (ROM), non-volatile RAM (NVRAM), electrically-erasable programmable ROM (EEPROM), flash memory, or any other digital media.

This disclosure describes techniques and devices configured to aid in the monitoring of the one or both kidneys of a patient. In some examples, processing circuitry 200 of external device 24 monitors the amount of oxygen dissolved in the urine (uPO₂) in the bladder as it has been shown that this measurement reflects the oxygenation of the kidneys. To do this the amount of oxygen dissolved in the urine may be sensed by the oxygen sensing element of sensor 21. In some examples, the urine output (rate of urine production) may also be sensed, for example by sensor 20. Example techniques of this disclosure utilize a catheter 10 with sensors to make these measurements. In some examples, the sensors are part of the catheter 10. In other examples, the sensors are not part of the catheter 10.

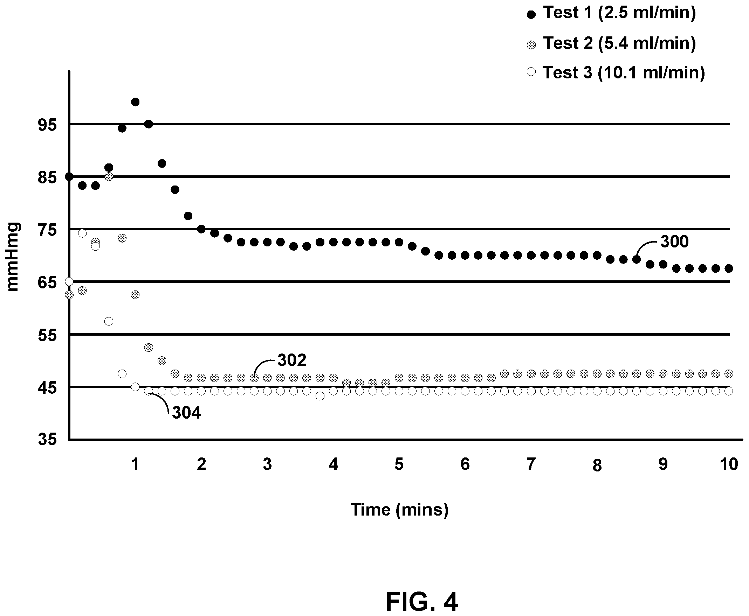

FIG. 4 is a graph illustrating pO₂ measurements of water that was initially set to ~43 mmHg and allowed to flow through a silicone Foley catheter at different flow rates. As shown in FIG. 4, the O₂ pick up from water with an initial pO₂ of ~43 mmHg flowing through a silicone Foley Catheter at a flow rate of 2.5 ml/min (Test 1 whose measurements shown as black filled circles 300) measured a pO₂ uptake of 26.3 mmHg. The O₂ pick up from water with an initial pO₂ of ~43 mmHg flowing through a silicone Foley Catheter at a flow rate of 5.4 ml/min (Test 2 whose measurements shown as grey filled circles 302) measured a pO₂ uptake of 6.4 mmHg. The O₂ pick up from water with an initial pO₂ of ~43 mmHg flowing through a silicone Foley Catheter at a flow rate of 10.1 ml/min (Test 3 whose measurements shown as white filled circles line 304) measured a pO₂ pick up of 3 mmHg. Some urine flow rates range from 0-5 ml/min for catheterized patients. Hence, the pO₂ pick up could be significant for silicone catheters.

Patients can be catheterized during and after major surgery using an indwelling urinary (Foley) catheter (e.g., catheter 10) inserted into the bladder via the urethra. Oxygen may be measured at the distal end of the inserted urinary catheter using an oxygen sensor (e.g., sensor 20) inserted in the flow stream between the catheter and the urine collecting bag. As mentioned above, commercially available Foley catheters are oxygen permeable in varying degree depending on the catheter material. This results in diffusion of oxygen between the urine in the catheter and the ambient air as well as the urethra over the catheter wall. Furthermore, the catheter wall constitutes an oxygen buffer which takes up/releases oxygen from/to the urine. These mechanisms can result in an alteration of the oxygen partial pressure (pO₂) from the true value in the bladder over the length of the catheter to the sample point at the distal end of the catheter where the pO₂ is measured. The degree of equilibration between the oxygen in the urine and the oxygen surrounding the catheter can be affected by: 1) the catheter material; 2) the inner and outer diameter of the catheter; 3) the wall thickness of the catheter; 4) the length of the catheter; 5) the portion of the catheter situated in the urethra and in the ambient air, respectively; 6) the flow speed of the urine - high flow speed results in a short transit time and thus, lower equilibration with the exterior oxygen concentration; 7) the change in flow speed; 8) the change in the oxygen partial pressure in the urine; and 9) temperature.

In general, silicone Foley catheters have a relatively high oxygen permeability resulting in a relatively high degree of oxygen equilibration with the oxygen at the outer wall of the catheter. Latex Foley catheters have a lower, but still potentially significant, oxygen equilibration with the oxygen at the outer wall of the catheter. PVC Foley catheters have the lowest oxygen permeability, but have the draw back that they are stiffer than the silicone and latex catheters, which may result in a lower degree of patient comfort and convenience.

Due to the relatively high oxygen permeability of Foley catheters, it may be desirable for measurement of urine oxygenation should be done as close to the kidneys as possible to obtain the best signal, e.g., more accurate reading indicative of the oxygenation status of the kidneys. However, it may not be easy to place a sensor through the ureter, so an alternative location to use is in the bladder. The measurements may also be taken outside of the body, but the urine transit through a Foley catheter has the possibility of changing the measurement. For example, silicone is a common material used in Foley catheters. Silicone has a relatively high permeability to oxygen. In some cases, the urine oxygenation is in the range of 10 to 50 mmHg, which is substantially lower than the atmospheric level of about 159 mmHg at sea level, creating a gradient that may drive atmospheric oxygen into the lumens of the Foley catheter. To make the most accurate measurement, it is preferable to take the measurement in the bladder.

FIG. 5 is a conceptual diagram of an example fiberoptic system according to the techniques of this disclosure. In some examples, a kidney monitoring system includes fiberoptic system 120. Fiberoptic system 120 may include oxygen sensing element 104 and/or temperature sensor 106 at or near one end (e.g., a proximal end), optical fiber 102, and processing circuitry 128 at or near the other end (e.g., a distal end). Oxygen sensing element 104 and/or temperature sensor 106 may be examples of sensor 21 and processing circuitry 128 may be an example of processing circuitry 28 (both of FIG. 1). Processing circuitry 128 may comprise electronic, optoelectronic, and/or optical components and be configured to process a signal(s) from oxygen sensing element 104 and/or temperature sensor 106. In some examples, fiberoptic system 120 may be partially insertable into lumen 32 (of FIG. 2) such that proximal portion 126B of fiberoptic system 120 may be received within proximal portion 17B of elongated body 12 (of FIG. 1), while processing circuitry 128 remains external to a patient. In some examples, fiberoptic system 120 may be partially housed within lumen 32 such that proximal portion 126B of fiberoptic system 120 may be housed within proximal portion 17B of elongated body 12 (of FIG. 1).

Optical fiber 102 may communicatively couple oxygen sensing element 104 and/or temperature sensor 106 to processing circuitry 128. When oxygen sensing element 104 is located within lumen 32 of catheter 10, oxygen sensing element 104 is configured to generate a signal indicative of the oxygen content of urine in a bladder of a patient without coming into directly contact with the urine. Oxygen sensing element 104 may be interrogated optically through optical fiber 102 which may be located in lumen 32 of the Foley catheter. In some examples, optical fiber 102 may be a plastic optical multimode fiber. Plastic optical multimode fiber may be more flexible and less brittle than a glass fiber and may have a higher numerical aperture thereby facilitating optical fiber 102 to acquire more light from oxygen sensing element 104. In some examples, processing circuitry 128 may be distal to the distal end of the catheter or may be located on the distal portion of the Foley catheter.

In some examples, optical fiber 102 has a suitable length for accessing a portion of lumen 34 (of FIG. 2) distal to anchoring member 18 (of FIG. 1). Optical fiber 102, oxygen sensing element 104 and temperature sensor 106 may have any appropriate size that may enable their insertion or location within lumen 34. In some examples, optical fiber 102, oxygen sensing element 104 and temperature sensor 106 are in the range of 100 microns to 1 millimeter and lumen 34 is larger than optical fiber 102, oxygen sensing element 104 and temperature sensor 106, but no larger than 3 millimeters.

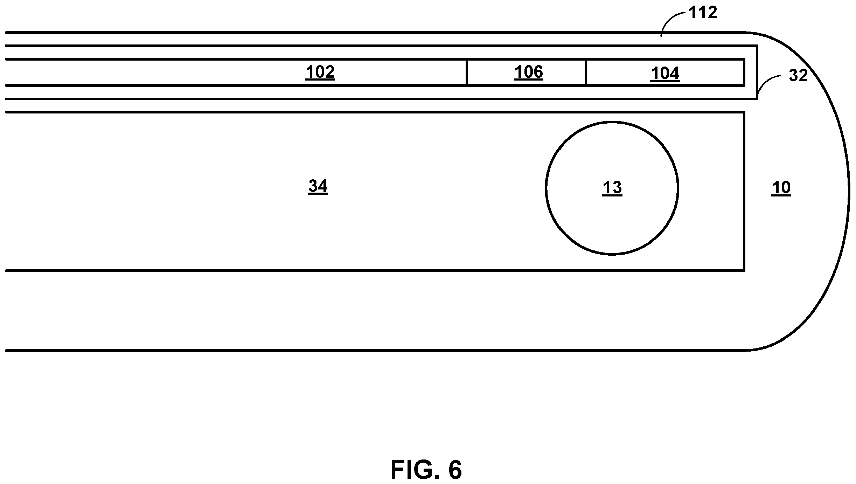

FIG. 6 is a conceptual diagram illustrating the proximal portion of an example fiberoptic system within a closed lumen 32 of an example catheter 10. Elongated body 12 of catheter 10 defines drainage lumen 34 having fluid opening 13 which may be configured to facilitate the inflow of a fluid, such as urine from the bladder of a patient, into drainage lumen 34. Elongated body 12 further defines lumen 32, which is closed at the proximal portion of catheter 10, such that elongated body 12 blocks the flow of the fluid from an environment (e.g., the bladder) external to the proximal portion of elongated body 12 into lumen 32. In accordance with the invention, oxygen sensing element 104 is located within lumen 32 of catheter 10. For example, oxygen sensing element 104 may be housed within lumen 32 or may be received within lumen 32 (e.g., oxygen sensing element 104 may be insertable into lumen 32). That is, lumen 32 is configured to house or receive oxygen sensing element 104.

Catheter 10 may be constructed of a largely oxygen permeable material like silicone or a material that may be less permeable to oxygen than silicone. In these examples, lumen 32, which may be a third lumen (e.g., not drainage lumen 34 or anchoring lumen 36 of FIG. 2) of a Foley catheter, that is closed. For example, elongated body 12 may be configured to block ingress of fluid into lumen 32 while oxygen sensing element 104 is positioned in lumen 32 and senses the amount of dissolved oxygen of fluid external to elongated body 12. In some examples, elongated body 12 may be configured to block oxygen sensing element 104 from directly contacting the fluid. For example, lumen 32, which may be a closed lumen, may effectively prohibit the flow of urine through lumen 32, thereby preventing oxygen sensing element 104 from being in contact with the urine, even when the Foley catheter is inserted in a patient and urine is flowing through fluid opening 13 through drainage lumen 34. Because the material separating lumen 32 from drainage lumen 34 and the bladder of the patient may be largely permeable to oxygen, oxygen sensing element 104 in lumen 34 may still be able to provide relatively accurate and responsive indications of oxygenation of urine in drainage lumen 34 or in the bladder without directly contacting such urine.

Oxygen sensing element 104 may be configured to sense an oxygen partial pressure in the urine of a patient. Oxygen sensing element 104 may sense the oxygen partial pressure in urine passing through drainage lumen 34 by sensing through the material separating lumen 32 and drainage lumen 34. Additionally, or alternatively, oxygen sensing element 104 may sense the oxygen partial pressure in urine in the bladder of the patient through outer wall 112 of catheter 10.

The catheter material between lumen 32 and the urine may act as a buffer, averaging out noise in the sensed oxygen content and delaying a response time for the measurement. Lumen 32 may be positioned within catheter 10 such that the wall thickness of the oxygen permeable material between oxygen sensing element 104 and the urine (e.g., in drainage lumen 34 or in the bladder of the patient) is at a desired value for a desired averaging and/or response time. For example, lumen 32 may be placed closer to drainage lumen 34, thereby facilitating a reduction in averaging and a faster response time when sensing the oxygen content of urine in drainage lumen 34 than when lumen 32 is placed further away from drainage lumen 34.

In some examples, another sensor may be included in lumen 34, such as, but not limited to, temperature sensor 106. For example, temperature may be sensed electronically using a thermocouple or a thermistor in lumen 34. In such examples, temperature sensor 106 may not be part of fiberoptic system 120 (of FIG. 5). In another example, temperature may be sensed using optical decay measurements by temperature sensor 106 as part of fiberoptic system 120. Similar to oxygen sensing element 104, temperature sensor 106 may be housed or received within lumen 34, which may be closed, and not in direct contact with the urine.

Due to the closed lumen environment and there being no physical contact with the urine by fiberoptic system 120, fiberoptic system 120 may be a re-usable component and reused for the same patient, with a different Foley catheter for the same patient, or with other patients. Fiberoptic system 120 may also be non-sterile. A device configuration with fiberoptic system 120 in a closed lumen may simplify the device by not having fiberoptic system 120 in contact with the urine or the bladder wall. This closed lumen may also be more effective at preventing unwanted leaks from the bladder of the patient than an open lumen.

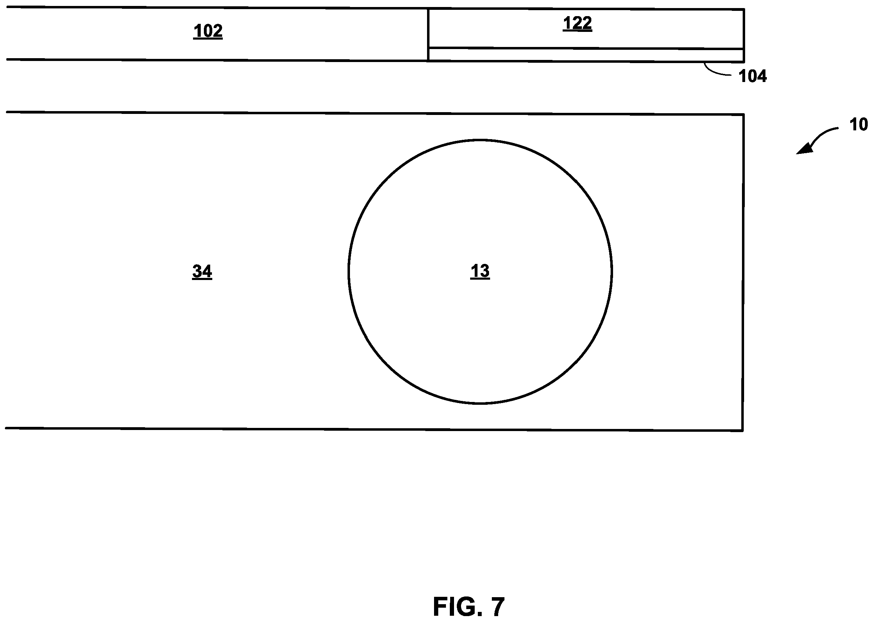

FIG. 7 is a conceptual diagram illustrating the proximal portion of an example fiberoptic system including a shield. Catheter 10 may include a closed lumen (similar to that of FIG. 6, but not shown for simplicity purposes), and optical fiber 102 having oxygen sensing element 104 may be inserted into or disposed within the closed lumen. However, in the example of FIG. 7, oxygen sensing element 104 may be partially enclosed by shield 122. In this manner, shield 122 may focus the area in which oxygen sensing element 104 may sense the oxygen partial pressure in the urine of the patient. For example, in the example of FIG. 7 shield 122 may shield the oxygen sensing element 104 from sensing oxygen through the outer wall (not shown) of catheter 10 and focus the oxygen sensing of the oxygen sensing element 104 towards urine flowing through drainage lumen 126. In some examples, rather than focus the oxygen sensing of oxygen sensing element 104 towards urine flowing through drainage lumen 34, shield 122 may be configured to focus the oxygen sensing of oxygen sensing element 104 towards an outer wall of catheter 10 and the urine in contact with the outer wall.

In some examples, shield 122 may cover the proximal end of oxygen sensing element 104. In some examples, shield 122 may be a separated mechanical part or, in other examples, may be a coating which may be directly applied onto oxygen sensing element 104. The opening of shield 122 may be directed towards a targeted surface of catheter 10 (e.g., an outer wall or drainage lumen 126). As in the example of FIG. 6, the closed lumen, in which optical fiber 102 and oxygen sensing element 104 may be located, may be positioned within catheter 10 such that the wall thickness of the oxygen permeable material between oxygen sensing element 104 and the urine is at a desired value for a desired averaging and/or response time.

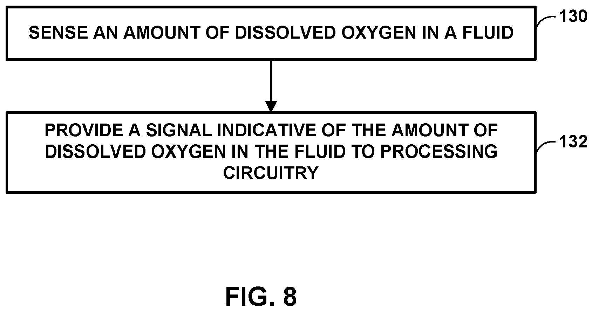

FIG. 8 is a flowchart illustrating example monitoring techniques. A clinician may introduce oxygen sensing element 104 into lumen 32 of catheter 10 or oxygen sensing element 104 can be housed in lumen 32 (e.g., pre-attached to elongated body 12 in lumen 32). Oxygen sensing element 104 senses an amount of dissolved oxygen in a fluid external to lumen 32 (130). For example, oxygen sensing element 104 may sense dissolved oxygen in urine in a bladder of a patient or in drainage lumen 34. Oxygen sensing element 104 may provide a signal indicative of the amount of dissolved oxygen in the fluid to processing circuitry 128 or to processing circuitry 200 of external device 24 (132). For example, oxygen sensing element 104 may provide a signal indicative of the amount of dissolved oxygen in the fluid to optical fiber 102. Optical fiber 102 may be configured to transport the signal, which may be an optical signal, from oxygen sensing element 104 to processing circuitry 128 and/or to processing circuitry 200 of external device 24. Oxygen sensing element 104 may be located in proximal portion 17B of lumen 32 of catheter 10. Catheter 10 may be configured to block ingress of the fluid into lumen 32 while oxygen sensing element 104 senses the amount of dissolved oxygen. Processing circuitry 128 or processing circuitry 200 of external device may be located at a distal portion of lumen 32 or distal to a distal end of the lumen 32.

In accordance with the invention, catheter 10 is configured to block oxygen sensing element 104 from directly contacting the fluid. According to the invention, lumen 34 is closed at a proximal end. In some examples, oxygen sensing element 104 is configured to measure optical decay.

In some examples, oxygen sensing element 114 is partially enclosed by shield 122. In some examples, shield 122 is configured to focus oxygen sensing by oxygen sensing element 114 towards an opening in shield 122. In some examples, temperature sensor 106 may sense a temperature of the fluid and provide a signal indicative of the temperature of the fluid processing circuitry 128 or processing circuitry 200 of external device 24. In some examples, temperature sensor 106 is located in proximal portion 17B of lumen 32 of catheter 10. In some examples, temperature sensor 106 comprises at least one of a thermocouple or a thermistor. In some examples, lumen 32 is a first lumen and catheter 10 comprises at least three lumens (e.g., drainage lumen 34, anchoring lumen 36 and lumen 32) including the first lumen.

Any of the techniques or examples described herein may be used alone or in combination with one or more other techniques or examples. These techniques may improve the ability more accurately sense oxygen content in a fluid than locating an oxygen sensor on a distal portion of a catheter or distal to a distal end of the catheter, as the oxygen content in the fluid may change as the fluid transits through the catheter. The techniques of this disclosure may provide an alternative location for a fiberoptic system than the drainage lumen of the Foley catheter. These techniques may improve the ability more accurately sense oxygen content in a fluid as drainage lumens may become clogged, for example, with blood, tissue, or other substances that may be in the bladder of the patient. Furthermore, by providing a location within the interior of the Foley catheter, there need not be an oxygen sensor element on body of catheter, thereby making the catheter easier to insert as there is only silicon in contact with the patient's urethra.

The techniques described in this disclosure, including those attributed to sensor 20, sensor 21, processing circuitry 200, communication circuitry 206, and UI 204 or various constituent components, may be implemented, at least in part, in hardware, software, firmware or any combination thereof. For example, various aspects of the techniques may be implemented within one or more processors, including one or more microprocessors, DSPs, ASICs, FPGAs, or any other equivalent integrated or discrete logic circuitry. The term "processor" or "processing circuitry" may generally refer to any of the foregoing logic circuitry, alone or in combination with other logic circuitry, or any other equivalent circuitry.

Such hardware, software, firmware may be implemented within the same device or within separate devices to support the various operations and functions described in this disclosure. In addition, any of the described units, modules or components may be implemented together or separately as discrete but interoperable logic devices. Depiction of different features as modules or units is intended to highlight different functional aspects and does not necessarily imply that such modules or units must be realized by separate hardware or software components. Rather, functionality associated with one or more modules or units may be performed by separate hardware or software components, or integrated within common or separate hardware or software components.

When implemented in software, the functionality ascribed to the systems, devices and techniques described in this disclosure may be embodied as instructions on a computer-readable medium such as RAM, ROM, NVRAM, EEPROM, FLASH memory, magnetic data storage media, optical data storage media, or the like. The instructions may be executed to support one or more aspects of the functionality described in this disclosure.

## Claims

1. A catheter (10) comprising:
an oxygen sensing element (21, 104); and
an elongated body (12) defining at least a first lumen (32) and a second lumen (34, 36), the first lumen (32) being configured to receive or house the oxygen sensing element (21, 104), wherein when the oxygen sensing element (21, 104) is located in a proximal portion (17B) of the first lumen (32), the oxygen sensing element (21, 104) is configured to sense an amount of dissolved oxygen in a fluid external to the first lumen (32),
**characterised in that**
the proximal portion (17B) of the first lumen (32) comprises a closed end such that the catheter (10) is configured to block ingress of the fluid into the first lumen (32) while the oxygen sensing element (21, 104) senses the amount of dissolved oxygen.

2. The catheter (10) of claim 1, wherein the catheter (10) comprises an outer wall (112) configured to separate the oxygen sensing element (21, 104) from the fluid when the oxygen sensing element (21, 104) is located in the first lumen (32).

3. The catheter (10) of claim 1 or claim 2, wherein the oxygen sensing element (21, 104) is configured to measure optical decay.

4. The catheter (10) of any combination of claims 1-3, wherein the oxygen sensing element (21, 104) is partially enclosed by a shield (122).

5. The catheter (10) of claim 4, wherein the shield (122) is configured to focus oxygen sensing by the oxygen sensing element (21, 104) towards an opening in the shield.

6. The catheter (10) of any combination of claims 1-5, further comprising:
a temperature sensor (106) configured to generate a signal indicative of a temperature of the fluid and to provide the signal to processing circuitry (28), wherein the temperature sensor (106) is located in the proximal portion of the first lumen (32).

7. The catheter (10) of claim 6, wherein the temperature sensor (106) comprises at least one of a thermocouple or a thermistor.

8. The catheter (10) of any combination of claims 1-7, wherein the elongated body (12) defines at least three lumens (32, 34, 36).

## Patentansprüche

1. Katheter (10), umfassend:
ein Sauerstofferfassungselement (21, 104); und
einen länglichen Körper (12), der mindestens ein erstes Lumen (32) und ein zweites Lumen (34, 36) definiert, wobei das erste Lumen (32) konfiguriert ist, um das Sauerstofferfassungselement (21, 104) aufzunehmen oder unterzubringen, wobei, wenn das Sauerstofferfassungselement (21, 104) sich in einem proximalen Abschnitt (17B) des ersten Lumens (32) befindet, das Sauerstofferfassungselement (21, 104) konfiguriert ist, um eine Menge an gelöstem Sauerstoff in einem Fluid außerhalb des ersten Lumens (32) zu erfassen, **dadurch gekennzeichnet, dass**
der proximale Abschnitt (17B) des ersten Lumens (32) ein geschlossenes Ende derart umfasst, dass der Katheter (10) konfiguriert ist, um ein Eindringen des Fluids in das erste Lumen (32) zu blockieren, während das Sauerstofferfassungselement (21, 104) die Menge an gelöstem Sauerstoff erfasst.

2. Katheter (10) nach Anspruch 1, wobei der Katheter (10) eine Außenwand (112) umfasst, die konfiguriert ist, um das Sauerstofferfassungselement (21, 104) von dem Fluid zu trennen, wenn das Sauerstofferfassungselement (21, 104) sich in dem ersten Lumen (32) befindet.

3. Katheter (10) nach Anspruch 1 oder 2, wobei das Sauerstofferfassungselement (21, 104) konfiguriert ist, um optischen Zerfall zu messen.

4. Katheter (10) gemäß einer beliebigen Kombination nach den Ansprüchen 1 bis 3, wobei das Sauerstofferfassungselement (21, 104) teilweise durch eine Abschirmung (122) umschlossen ist.

5. Katheter (10) nach Anspruch 4, wobei die Abschirmung (122) konfiguriert ist, um die Sauerstofferfassung durch das Sauerstofferfassungselement (21, 104) in Richtung einer Öffnung in der Abschirmung zu fokussieren.

6. Katheter (10) gemäß einer Kombination nach den Ansprüchen 1 bis 5, ferner umfassend:
einen Temperatursensor (106), der konfiguriert ist, um ein Signal zu erzeugen, das eine Temperatur des Fluids angibt, und um das Signal einer Verarbeitungsschaltung (28) bereitzustellen, wobei der Temperatursensor (106) sich in dem proximalen Abschnitt des ersten Lumens (32) befindet.

7. Katheter (10) nach Anspruch 6, wobei der Temperatursensor (106) mindestens eines von einem Thermoelement oder einem Thermistor umfasst.

8. Katheter (10) gemäß einer Kombination nach den Ansprüchen 1 bis 7, wobei der längliche Körper (12) mindestens drei Lumen (32, 34, 36) definiert.

## Revendications

1. Cathéter (10) comprenant :
un élément de détection d'oxygène (21, 104) ; et
un corps allongé (12) définissant au moins une première lumière (32) et une seconde lumière (34, 36), la première lumière (32) étant conçue pour recevoir ou loger l'élément de détection d'oxygène (21, 104), dans lequel, lorsque l'élément de détection d'oxygène (21, 104) est situé dans une partie proximale (17B) de la première lumière (32), l'élément de détection d'oxygène (21, 104) est configuré pour détecter une quantité d'oxygène dissous dans un fluide extérieur à la première lumière (32), **caractérisé en ce que**
la partie proximale (17B) de la première lumière (32) comprend une extrémité fermée de telle sorte que le cathéter (10) est conçu pour bloquer l'entrée du fluide dans la première lumière (32) pendant que l'élément de détection d'oxygène (21, 104) détecte la quantité d'oxygène dissous.

2. Cathéter (10) selon la revendication 1, dans lequel le cathéter (10) comprend une paroi extérieure (112) conçue pour séparer l'élément de détection d'oxygène (21, 104) du fluide lorsque l'élément de détection d'oxygène (21, 104) est situé dans la première lumière (32).

3. Cathéter (10) selon la revendication 1 ou la revendication 2, dans lequel l'élément de détection d'oxygène (21, 104) est configuré pour mesurer la décroissance optique.

4. Cathéter (10) selon l'une quelconque combinaison des revendications 1-3, dans lequel l'élément de détection d'oxygène (21, 104) est partiellement entouré d'un bouclier (122).

5. Cathéter (10) selon la revendication 4, dans lequel le bouclier (122) est conçu pour concentrer la détection d'oxygène par l'élément de détection d'oxygène (21, 104) vers une ouverture dans le bouclier.

6. Cathéter (10) selon l'une quelconque combinaison des revendications 1-5, comprenant en outre :
un capteur de température (106) configuré pour générer un signal indiquant une température du fluide et pour fournir le signal au circuit de traitement (28), dans lequel le capteur de température (106) est situé dans la partie proximale de la première lumière (32).

7. Cathéter (10) selon la revendication 6, dans lequel le capteur de température (106) comprend au moins l'un parmi un thermocouple ou une thermistance.

8. Cathéter (10) selon l'une quelconque combinaison des revendications 1-7, dans lequel le corps allongé (12) définit au moins trois lumières (32, 34, 36).
